# EUROPEAN PATENT APPLICATION

(11) **EP 2 848 286 A1**
(43) Date of publication of application: **18.03.2015**
(21) Application number: 13184149.6
(22) Date of filing: 12.09.2013
(51) Int. Cl.: A61Q 19/06, A61Q 19/08, A61K 8/35, A61K 8/365, A61K 8/97, A61K 36/28

(54) **Cosmetic composition comprising chicoric acid**

(71) Applicant: DSM IP Assets B.V., 6411 TE Heerlen (NL)
(72) Inventor: The designation of the inventor has not yet been filed
(74) Representative: Barthelemy de Saizieu, Antoine

(57) **Abstract**

The present invention relates to the use of a cosmetic composition comprising chicoric acid for the prevention and/or treatment of skin fatty deposits and cellulite in human. Said chicoric acid is more particularly suited for the prevention and/or treatment of skin fatty deposits and cellulite in human characterized by a skin having an orange-peel texture.

## Description

The present invention relates to the use of a cosmetic composition comprising chicoric acid for reducing and/or eliminating skin fatty deposits and cellulite characterized by orange peel appearance, in human, and/or skin firming, and/or for refining contours of the face, and/or for treatment of weight overloads of the thighs and the hips. It also relates to a cosmetic composition comprising chicoric acid, and to a method of reducing and/or eliminating skin fatty deposits and cellulite in human, and/or skin firming, and/or for refining the contours of the face, comprising topically applying to the skin an effective amount of a cosmetic composition comprising chicoric acid, and observing the result.

Cellulite was first described in the seventeenth century, but the term first began to appear in literature only in the 70's regarding skin appearance. There is a growing demand in the industry, and a growing market need for cosmetic skin care products that prevent, delay the appearance, or reduce skin fatty deposits and cellulite in human.

Not all cellulite looks the same, and there is a spectrum of the disorder that can be divided into five grades according to the classification of Rossi and Vergnanini 2000, JEADV 14, 251-22. The presence of cellulite is an aesthetically unacceptable cosmetic problem affecting most post-pubescent females. It is largely observed in the gluteal-femoral regions characterized with its "orange peel, or "cottage cheese" appearance. Cellulite has been described as a normal condition that maximizes subcutaneous fat retention to ensure adequate caloric availability for pregnancy and lactation. It is thus considered by the medical community not as a clinical entity, but as a normal variant of fat distribution in women.

Cellulite is attenuated with massage which decreases tissue oedema, and also by topical creams which improve aesthetic appearance of the skin such as xanthines, botanicals, fragrances, and ligands for the retinoid and peroxisomal proliferator-activated receptors (Rawlings 2006, Int. J. Cosmet. Sci. 28 175-170). However, such topical compositions described in the prior art are not or poorly efficacious, have toxic side effects when used at effective dose, and are expensive for the consumer in need of improving the aesthetic appearance of the skin.

Surprisingly, we have now found out that chicoric acid is able to very efficiently suppress formation of lipid droplets in adipocytes thereby leading to a very strong effect in reducing and/or eliminating skin fatty deposits and cellulite in human skin, as well as in stimulating skin firming, and/or for refining contours of the face, and/or for treatment of weight overloads of the thighs and the hips.

Therefore, the first object of the present invention is the use of a topical cosmetic composition comprising chicoric acid for reducing and/or eliminating skin fatty deposits and cellulite, and/or for skin firming, and/or for refining contours of the face and ameliorating the aesthetic appearance of the skin in human.

It is also an object of the present invention to provide a topical cosmetic composition comprising chicoric acid for use in the prevention and treatment of skin fatty deposits and cellulite in human and/or for treatment of weight overloads of the thighs and the hips.

The use according to the present invention as described above includes prevention and/or reduction of all outward visibly and tactilely perceptible manifestations as well as any other macro or micro symptoms notices on the skin due to cellulite. In a later stage, cellulite is characterized by with its "orange peel, or "cottage cheese" appearance. The present invention has been found to act on both man and women. Therefore, it can be used topically on any human tissue characterized by an excess weight like weight overload of thighs, and or hips.

The term "cosmetic composition" in accordance with the present invention relates to a formulation that can be used for cosmetic purposes, purposes of hygiene, or as a basis for delivery of one or more pharmaceutical or cosmetic ingredients.

Chicoric acid (CAS 70831-56-0) is a caffeic acid derivative, belonging to the group of polyphenols and is represented by the following formula (I).

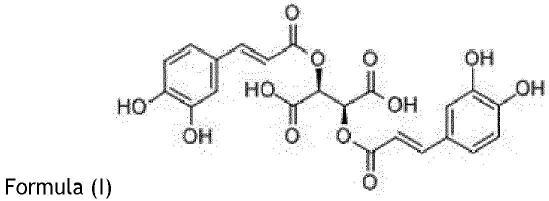

Chicoric acid as used in the present invention can be extracted from the aerial parts (leaves and flowers) of plants of the genus *echinacea.* Preferably, it is extracted from *Echinacea purpurea* or obtained in a pure form from a number of chemical supplyers (e.g.: Chromadex US or Extrasynthese, France).

In another embodiment, the topical cosmetic composition according to the present invention comprises between 0.0001 and 3 weight-% chicoric acid in dry matter. More preferably, the cosmetic composition comprises between 0.001 and 1 weight-%, even more preferably, it comprises between 0.01 and 0.5 weight-%, chicoric acid.

According to the present invention, chicoric acid can be used as such in a topical cosmetic composition or in an encapsulated form, for example in a liposomal form. Liposomes are preferably formed with lecithins with or without addition of sterols or phytosterols. The encapsulation of the active ingredient can be alone or together with other active ingredients. Other embodiments include solid or semisolid capsules aiming to protect the chicoric acid from degradation or for controlled delivery. Suitable encapsulation technologies are for example described in WO 0180823, WO 9903450, WO 9317784 or in Fragrance Journal (2001), 29(2), 83-90.

Preferred topical cosmetic composition according to the present invention is a topical composition further comprising a conventional cosmetic carrier. More preferably, it is a skin (face and body) care preparation, a decorative preparation, a light protection preparation, or a functional preparation.

Examples of skin care preparations are, in particular, face creams, body oils, body lotions, body gels, treatment creams, skin protection ointments, shaving preparations, such as shaving foams or gels, moisturizing gels, moisturizing sprays, revitalizing body sprays, cellulite gels, face and/or body moisturizers, facial and/or body cleansers, and face masks. Most preferred are face care products.

Preferred topical cosmetic compositions according to the invention are skin care preparations, or functional preparations.

Examples of decorative preparations are, in particular, lipsticks, eye shadows, mascaras, dry and moist make-up formulations, rouges, powders, and/or suntan lotions.

Examples of functional preparations are cosmetic compositions containing further active ingredients such as hormones, vitamins, vegetable and/or fruit extracts, anti-ageing ingredients, and/or antimicrobial (antibacterial or antifungal) ingredients without being limited thereto.

Topical cosmetic composition for use in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foams, sprays, sticks, a gel, a plaster, a powder, a cleanser, a soap or aerosols or wipes. Preferred topical compositions comprise a cream, an emulsion, a gel, an ointment, a lotion a tincture, a spray, a mousse, a cleansing composition or foam.

In another embodiment, the topical cosmetic composition for use according to the present invention is **characterized in that** it further comprises at least one UV screening agent, and/or a moisturizer, and/or an anti-aging agent, and/or a skin tone agent, and a conventional cosmetic carrier.

Preferably, the topical composition for use according to the present invention is **characterized in that** it comprises a topical anti cellulite agent.

Conventional cosmetic carriers comprise excipients or diluents conventionally used in topical compositions. If nothing else is stated, the excipients, additives, diluents, etc. mentioned in the following are suitable for both pharmaceutical and cosmetic compositions. The necessary amounts of the cosmetic and dermatological adjuvants and additives can, based on the desired product, easily be determined by the skilled person.

Regarding the kind of the topical cosmetic composition and the preparation of the topical cosmetic preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

Preferably, the topical cosmetic compositions used in the present invention are in the form of a suspension or dispersion in solvents or fatty substances, or alternatively in the form of an emulsion or micro emulsion (in particular of O/W or W/O type, O/W/O or W/O/W-type), PET-emulsions, multiple emulsions, bickering emulsions, hydrogels, alcoholic gels, lipogels, one or multiphase solutions or a vesicular dispersion and other usual compositions, which can also be applied by pens, as masks or as sprays. The emulsions can also contain anionic, nonionic, cationic or amphoteric surfactant(s).

The topical cosmetic or pharmaceutical compositions used in the present invention can also contain usual cosmetic or pharmaceutical adjuvants and additives, such as preservatives/ antioxidants, fatty substances/ oils, water, organic solvents, silicones, thickeners, softeners, emulsifiers, sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photo-protective effect by physically blocking out ultraviolet radiation, or any other ingredients usually formulated into topical cosmetic or pharmaceutical compositions. The necessary amounts of the cosmetic and dermatological adjuvants and additives can, based on the desired product, easily be chosen by a skilled artisan in this field and will be illustrated in the examples, without being limited hereto. The usual cosmetic adjuvants and additives such as emulsifiers, thickeners, surface active ingredients and film formers can show synergistic which can be determined by the expert in the field with normal trials, or with the usual considerations regarding the formulation of topical cosmetic or pharmaceutical composition.

Typically topical cosmetic or pharmaceutical compositions also contain surface active ingredients like emulsifiers, solubilizers and the like. An emulsifier enables two or more immiscible components to be combined homogeneously. Moreover, the emulsifier acts to stabilize the composition. Solubilizers that may be used in the present invention include but are not restricted to PEG/PPG-18/18 Dimethicone, PEG-40 Hydrogenated Castor Oil, PEG-20 Stearate, PEG-30 Glyceryl Stearate, and PEG-7 Glyceryl Cocoate. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4- oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the topical cosmetic or pharmaceutical topical composition for use in the present invention. Preferably, about 0.1 wt.% to about 10 wt.% of emulsifiers is used.

The lipid phase of the topical cosmetic or pharmaceutical compositions can advantageously be chosen from: mineral oils and mineral waxes; oils such as triglycerides of caprinic acid or caprylic acid and castor oil; oils or waxes and other natural or synthetic oils, in a preferred embodiment esters of fatty acids with alcohols e.g. isopropanol, propylene glycol, glycerin or esters of fatty alcohols with carboxylic acids or fatty acids; alkylbenzoates; and/or silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the topical cosmetic or pharmaceutical composition of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleate, isooctylstearate, isononylstearate, isononylisononanoate, 2- ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical cosmetic or pharmaceutical compositions for use according to the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicones (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical cosmetic or pharmaceutical compositions for use according to the present invention are isoeikosane; neopentylglycoldiheptanoate; propyleneglycoldicaprylate/ dicaprate; caprylic/ capric/ diglycerylsuccinate; butyleneglycol caprylat/caprat; C₁₂₋₁₃-alkyllactate; di-C₁₂₋₁₃ alkyltartrate; triisostearin; dipentaerythrityl hexacaprylat/hexacaprate; propyleneglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the topical cosmetic or pharmaceutical compositions for use according to the present invention can also contain natural vegetable or animal waxes such as bee wax, china wax, bumblebee wax and other waxes of insects as well as Shea butter and cocoa butter.

A moisturizing agent may be incorporated into a topical cosmetic or pharmaceutical composition used according to the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅- alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the topical cosmetic or pharmaceutical composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a topical cosmetic or pharmaceutical composition of the present invention such as glycerin, polypropylene glycol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a topical cosmetic or pharmaceutical composition of the present invention, preferably about 1 wt.% to about 5 wt.%.

The aqueous phase of the preferred topical cosmetic or pharmaceutical compositions for use in the present invention can contain the usual cosmetic or pharmaceutical additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or-monobutylether, diethyleneglycol monomethyl-or monoethylether and analogue products, polymers, foam stabilizers; electrolytes and especially one or more thickeners. However, preferably the topical cosmetic or pharmaceutical compositions for use in the present invention are free of ethanol, more preferably they are free of alcohols, and most preferably they are free of organic solvents, since such compounds can cause skin irritation.

Thickeners that may be used in topical cosmetic or pharmaceutical compositions for use in the present invention to assist in making the consistency of a product suitable include carbomer, siliciumdioxide, magnesium and/ or aluminium silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof.

Suitable neutralizing agents which may be included in the topical cosmetic or pharmaceutical composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the topical cosmetic or pharmaceutical composition of the present invention, preferably, 1 wt.% to about 5 wt.%.

The addition of electrolytes into the topical cosmetic or pharmaceutical composition for use according to the present invention may be necessary to change the behavior of a hydrophobic emulsifier. Thus, the emulsions/ microemulsions for use according to this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt. % to about 8 wt. % in the topical cosmetic or pharmaceutical composition of the present invention.

The use of the topical cosmetic or pharmaceutical compositions according to the present invention is preferably performed by application at least once per day, e.g. twice or triple times a day on the skin, preferably on the face.

Furthermore, the present invention relates to a method of reducing and/or eliminating of skin fatty deposits and cellulite in human, and/or skin firming, and/or for refining the contours of the face, comprising topically applying an effective amount of a cosmetic composition comprising chicoric acid, and observing the result.

The term 'an effective amount' refers to an amount necessary to obtain a physiological effect. The physiological effect may be achieved by one application dose or by repeated applications. The dosage administered may, of course, vary depending upon known factors, such as the frequency of treatment; and the effect desired and can be adjusted by a person skilled in the art.

The present invention also relates to a method as described above, wherein, from about 0.2 µg to about 200 µg of chicoric acid, is applied per square centimeter of skin per day.

The usefulness of agents in reducing and/or eliminating of skin fatty deposits and cellulite in human, and/or skin firming, and/or for refining the contours of the face can be determined by methods known in the art. For example, Cellulite condition can be visually evaluated by basic expert grading and also after standardized pinching of the skin to evaluate the orange peel appearance of the skin surface. The circumference of the upper thighs can be measured. The dermal torquemeter can be used to measure skin tonicity. Reconstructed volume of the thigh, hip and buttock between two fixed horizontal slices can be measured with 3D Fringe projection and 3D reconstruction images. This technique can also be used to evaluate facial contours. DermaScan ultrasound imaging is also possible for non-invasive evaluation of dermal- and subcutaneous changes in the tissue structure.

The following Example is illustrative but not limitative of the invention.

### Examples

### Example 1: Adipocyte differentiation in the presence of chicoric acid

### Adipocyte differentiation:

C3H10 T1/2 cells were seeded on Collagen Type I coated 24-well plates at a cell density of 3.8x10⁴ cells/mL in Differentiation Base medium (DM-Base), containing DMEM without Phenol Red, supplemented with 10% FCS, 4mM L-Glutamine and 1% Pen/Strep (v/v) at 37° C in an incubator with 5% CO₂ atmosphere and 85%-90% relative humidity over 72hours. After this pre-incubation, DM-Base is replaced by DM-Supplemented, containing DM-Base plus 200nM Insulin, plus 10µM Rosiglitazone. Test compounds (e.g.: chicoric acid) were added in DM-Supplemented, and kept in culture for 7 consecutive days. Each experiment was performed in triplicates. Every second day medium and compounds were renewed.

### Staining Procedure :

Cells were fixed by adding 1mL of 60% 2-Propanot in PBS to each well and incubated for 1.5h at 4°C. The cells were then twice washed with 1mL PBS/well. Staining was performed in one step and 300µL/well of fluorescent dyes Hoechst 33342 (Hoe) and Bodipy® 493/503 (BP) were added to each well. Stock solutions of Hoe (10mg/mL in PBS) and BP (1mg/mL in PBS) were diluted with PBS, a dilution of 1:5000 for Hoe and 1:4000 for BP was applied. The plate was then incubated at RT in the dark for 30 minutes. Before analysis, the cells were washed once with PBS, leaving the cells covered with 1mL/well of PBS, and the plate wrapped in aluminium foil and stored at 4°C in the dark.

### Fluorescence Microscopy / Automated determination of lipid accumulation in adipocytes:

Lipid droplets were quantified with the Cellomics® Array Scan® VTI HCS Reader. Fat droplets were detected with the provided SpotDetector® Bio-Application. The fluorescent dye, BODIPY® 493/503, was used to visualise and quantify fat droplets (number, area, intensity) in differentiated C3H10 T1/2 adipocytes. Nuclei were stained using the dye Hoechst 33342. The method is based on a two-channel assay, which uses a 40x objective, a Hamamatsu ORCA-ER digital camera in combination with a 0.63x coupler and Carl Zeiss microscope optics for image acquisition. Images were acquired in high resolution (1024 x 1024, 1 x 1) and auto focus mode (1024 x 1024, 4 x 4) resulting in a field width of 262 microns (pixel size). Channel one (Ch1) applies the XF93-Hoechst filter and is the focus channel in which objects (nuclei) are identified, whereas the spots (fat droplets) are detected in channel two (Ch2) using a XF93-FITC filter.

### Results :

Chicoric acid was tested at three different concentrations (2, 10 and 50 micro molar). At 50 micromolar concentration, chicoric acid produced a very strong 69% decrease in the average number of lipid droplets per adipocyte as well as a 42 % decrease in the average size of the lipid droplets in adipocytes.

## Claims

1. Use of a topical cosmetic composition comprising chicoric acid for reducing and/or eliminating skin fatty deposits and cellulite, and/or for skin firming, and/or for refining contours of the face and ameliorating the aesthetic appearance of the skin in human.

2. A topical cosmetic composition comprising chicoric acid for use in the prevention and treatment of skin fatty deposits and cellulite in human, and/or for treatment of weight overloads of the thighs and the hips.

3. The use according to claim 1 or 2, wherein chicoric acid is L-chicoric acid.

4. The use according to any of the claims 1 to 3, wherein the cosmetic composition comprises between 0.0001 and 3 weight-% chicoric acid.

5. The use according to any of the claims 1 to 4, wherein the cosmetic composition comprises between 0.01 and 1 weight-% chicoric acid.

6. The use according to any of the claims 1 to 4, wherein the topical cosmetic composition is in the form of a cream, an emulsion, a gel, an ointment, a lotion, a tincture, a spray, a mousse, a cleansing composition or a foam.

7. The use according to any of the claims 1 to 6, wherein the composition further comprises a topical anti-cellulite agent.

8. The use according to any of the claims 1 to 7, wherein the cosmetic composition further comprises a conventional cosmetic or pharmaceutical carrier.

9. Topical cosmetic composition comprising chicoric acid and a conventional cosmetic carrier.

10. Method of reducing and/or eliminating of skin fatty deposits and cellulite in human, and/or skin firming, and/or for refining the contours of the face, comprising topically applying an effective amount of a cosmetic composition comprising chicoric acid, and observing the result.
